# EUROPÄISCHE PATENTSCHRIFT

(11) **EP 2 303 743 B1**
(45) Veröffentlichungstag und Bekanntmachung des Hinweises auf die Patenterteilung: **19.09.2012**
(21) Anmeldenummer: 09799915.5
(22) Anmeldetag: 14.07.2009
(51) Int. Cl.: B65H 63/06, D01H 13/22, D02G 3/34, G01N 33/36, G01N 21/89

(54) **VERFAHREN UND VORRICHTUNG ZUR GARNREINIGUNG**
METHOD AND DEVICE FOR YARN CLEANING
PROCÉDÉ ET DISPOSITIF DE NETTOYAGE DE FIL

(30) Priorität: 25.07.2008 CH 11652008
(43) Veröffentlichungstag der Anmeldung: 06.04.2011
(73) Patentinhaber: Uster Technologies AG, 8610 Uster (CH)
(72) Erfinder: SOELL, Wolfram, CH-8610 Uster (CH); NARAYANAN, Sivakumar, CH-8610 Uster (CH); SCHMID, Peter, CH-8050 Zürich (CH); SCHNEIDER, Ulf, CH-8610 Uster (CH); NASIOU, Thomas, CH-8610 Uster (CH)
(86) Internationale Anmeldenummer: PCT/CH2009/000254
(87) Internationale Veröffentlichungsnummer: WO 2010/009565

(56) Entgegenhaltungen:
- WO-A-01/92875
- WO-A-97/36032
- WO-A-2005/042815
- WO-A-2007/056883
- US-B1- 6 374 152

## Beschreibung

### FACHGEBIET

Die vorliegende Erfindung betrifft ein Verfahren und eine Vorrichtung zur Ausreinigung von Fehlern aus einem in einer Textilmaschine entlang seiner Längsrichtung bewegten Garn, gemäss den Oberbegriffen der unabhängigen Patentansprüche. Ihr bevorzugter Einsatz ist auf Spinn- oder Spulmaschinen. Die Erfindung eignet sich für die Reinigung von Garnen und Fehlern aller Arten, besonders aber für die Ausreinigung von Massen-oder Dickenungleichmässigkeiten aus einem Effektgarn.

### STAND DER TECHNIK

Garnreiniger werden an Textilmaschinen wie Spinn- oder Spulmaschinen zur Qualitätskontrolle am laufenden Garn eingesetzt. Ein Garnreiniger beinhaltet mindestens eine Messzelle mit einem Gamsensor zur Erfassung einer Garneigenschaft und eine Auswerteeinheit zur Beurteilung, ob die detektierten Garneigenschaften bestimmten Qualitätskriterien genügen oder nicht. Fakultativ kann der Garnreiniger auch eine Schneideinrichtung zur Entfernung qualitativ ungenügender Garnabschnitte enthalten. Die Qualitätskriterien werden üblicherweise durch eine Reinigungsgrenze vorgegeben. Dies ist eine Linie in einem Quadranten eines zweidimensionalen kartesischen Koordinatensystems, in dem die Amplituden gegenüber den Längen der detektierten Garnfehler eingetragen werden. Die Reinigungsgrenze teilt den Quadranten in zwei zueinander komplementäre Bereiche auf. Garnfehler diesseits der Reinigungsgrenze werden toleriert, Garnfehler jenseits der Reinigungsgrenze herausgeschnitten oder zumindest als Fehler registriert. Die Reinigungsgrenze kann einen unstetigen, stufenförmigen oder einen stetigen Verlauf haben. Mit der Festlegung der Reinigungsgrenze befasst sich z. B. die US-6,374,152 B1.

Dem genannten kartesischen Koordinatensystem ist üblicherweise eine Klassiermatrix überlagert, welche aus einer Vielzahl von rechteckigen, durch achsparallele Geraden voneinander abgegrenzten Klassen besteht. Gemäss der US-4,169,399 A folgt die stufenförmige Reinigungsgrenze den Klassengrenzen der Klassiermatrix.

Die WO-97/36032 A1 lehrt, optisch detektierte Fremdstoffe in einem Garn gemäss ihrer Reflektivität und Längenausdehnung in einer Klassiermatrix zu klassieren und je nach ihrer Klassenzugehörigkeit auszusondern. Die Fremdstoffklassen sind dementsprechend immer rechteckig, und ihre Lage ist nicht beliebig, sondern durch das Raster der achsparallelen Geraden vorgegeben. Mit einer eigentlichen Reinigungsgrenze, wie sie die US-6,374,152 B1 offenbart, befasst sich die WO-97/36032 A1 nicht.

Effektgarn ist ein besonderes Garn, dessen Struktur oder Faserzusammensetzung von normalem Glattgarn abweicht. Es wird als bereicherndes Element in Weberei- und Strickereiprodukten eingesetzt. Üblicherweise weist Effektgarn eine Vielzahl von Dickstellen oder Dünnstellen - so genannten Effekten ― auf, deren Durchmesser deutlich grösser bzw. kleiner ist als der Durchmesser der zwischen zwei Effekten liegenden Garnabschnitte ― der so genannten Stege. Verfahren und Vorrichtungen zur Herstellung und Charakterisierung von Effektgarn sind bekannt, z. B. aus den Veröffentlichungen WO-2005/037699 A1, WO-2005/038105 A1, WO-2005/042815 A2, WO-2007/056883 A2, WO-2007/056884 A2 und WO-2007/056885 A2. Keine dieser Veröffentlichungen befasst sich mit der Reinigung von Effektgarn.

Die Reinigung von Effektgarn ist deshalb schwierig, weil ohne besondere Massnahmen Effekte für störende Fehler gehalten und ausgereinigt werden. Ein Verfahren und eine Vorrichtung zur Reinigung von Effektgarn sind aus der WO-2005/047155 A1 bekannt. Dabei sollen einerseits die Werte für Durchmesser oder Masse der Stege gemessen und mit einer oberen und einer unteren Reinigungsgrenze verglichen werden. Andererseits sollen auch die Werte für Durchmesser oder Masse der Effekte ebenfalls mit einer oberen und einer unteren Reinigungsgrenze verglichen werden. Die beiden Reinigungsgrenzenpaare begrenzen je einen Bereich für Werte von Stegen und Effekten, in denen zulässige Messwerte liegen sollen. Messwerte, die jenseits dieser beiden Bereiche liegen, zeigen Fehler im Garn an. Für die letztgenannten Fehler jenseits der Reinigungsgrenzen können zusätzlich ihre Längen mit einbezogen werden, indem genügend kurze Fehler nicht ausgereinigt werden, selbst wenn sie jenseits der Reinigungsgrenzen liegen. Ein Nachteil des Verfahrens und der Vorrichtung gemäss der WO-2005/047155 A1 besteht darin, dass ihre Wirksamkeit und ihre Möglichkeiten, die Reinigung des Effektgarns zu gestalten, sehr beschränkt sind. Sie eignen sich nur für Effektgarn mit genau einem vorgegebenen Effektdurchmesser und können Effekte mit unterschiedlichen Durchmessern kaum verarbeiten. Die Effektlänge berücksichtigen sie überhaupt nicht, so dass zu kurze und zu lange Effekte sowie Garnfehler im Stegbereich, deren Durchmesser zufällig mit dem Effektdurchmesser übereinstimmt, nicht erkannt werden.

### DARSTELLUNG DER ERFINDUNG

Es ist eine Aufgabe der vorliegenden Erfindung, eine verfeinerte Ausreinigung von Fehlern aus Garnen, insbesondere aus Effektgarnen, zu ermöglichen. Spezielle Charakteristika der Garne, wie z. B. die Charakteristika von Effekten bei Effektgarnen, sollen weitergehend berücksichtigt werden als im Stand der Technik.

Diese und andere Aufgaben werden durch das erfindungsgemässe Verfahren und die erfindungsgemässe Vorrichtung gelöst, wie sie in den unabhängigen Patentansprüchen definiert sind. Vorteilhafte Ausführungsformen sind in den abhängigen Patentansprüchen angegeben.

Unter dem Begriff "Ereignisfeld" wird in dieser Schrift ein Quadrant eines zweidimensionalen kartesischen Koordinatensystems, oder ein Teil eines solchen Quadranten, verstanden, dessen Abszisse eine Erstreckung von Garnparameterwerten in der Längsrichtung und dessen Ordinate eine Abweichung eines Garnparameters von einem Sollwert angibt. Der Garnparameter kann z. B. die Garnmasse pro Längeneinheit oder der Garndurchmesser sein, und dementsprechend ist der Sollwert z. B. eine Soll-Garnmasse pro Längeneinheit oder ein Soll-Garndurchmesser. Punkte in diesem Ereignisfeld werden "Garnereignisse" oder einfach "Ereignisse" genannt.

Die Erfindung beruht auf der Idee, im Ereignisfeld zusätzlich zu den beiden durch eine Reinigungsgrenze voneinander abgegrenzten Bereichen weitere Bereiche zu definieren, die zulässige oder unzulässige Ereignisse vorgeben. Ein Beispiel für einen solchen weiteren, dritten Bereich ist ein "Reinigungsfenster", das einen Bereich für zu tolerierende Effekte eines Effektgarns im an sich unzulässigen Bereich jenseits der Reinigungsgrenze vorgibt.

Im erfindungsgemässen Verfahren zur Ausreinigung von Fehlern aus einem in einer Textilmaschine, bspw. einer Spinnmaschine oder einer Spulmaschine, entlang seiner Längsrichtung bewegten Garn werden Messwerte einer Eigenschaft des Garns entlang der Längsrichtung des Garns erfasst. Werte eines Garnparameters werden aus den Messwerten ermittelt. Es wird ein Ereignisfeld bereitgestellt, das ein Quadrant oder ein Teil eines Quadranten eines zweidimensionalen kartesischen Koordinatensystems ist, dessen Abszisse eine Erstreckung von Garnparameterwerten in der Längsrichtung und dessen Ordinate eine Abweichung des Garnparameters von einem Sollwert angibt. Das Ereignisfeld wird in Bereiche aufgeteilt, von denen ein erster Bereich, der an die Abszisse sowie an die Ordinate angrenzt und ferner durch eine Reinigungsgrenze begrenzt ist, zulässige Ereignisse und ein zum ersten Bereich komplementärer zweiter Bereich unzulässige Ereignisse vorgibt. Die Werte des Garnparameters werden mitsamt ihrer Erstreckung in der Längsrichtung in dem Ereignisfeld klassiert. Im Ereignisfeld wird mindestens ein weiterer Bereich definiert, der im ersten Bereich unzulässige Ereignisse oder im zweiten Bereich zulässige Ereignisse vorgibt.

Die Formulierung, wonach der erste Bereich "an die Abszisse sowie an die Ordinate angrenzt", lässt offen, ob der erste Bereich direkt von der betreffenden Achse begrenzt ist oder ob eine grafische Darstellung gewählt wird, in der ein Abstand zwischen der betreffenden Achse und dem ersten Bereich vorhanden ist. Wesentlich ist, dass sich zwischen der betreffenden Achse und dem ersten Bereich kein anderer Bereich befindet, der zulässige oder unzulässige Ereignisse vorgibt.

Der weitere Bereich kann vom ersten Bereich oder vom zweiten Bereich im Wesentlichen umschlossen sein. Für eine solche Ausführungsform trifft zu, dass von jeweils zwei aneinander grenzenden Bereichen der eine Bereich zulässige Ereignisse und der andere Bereich unzulässige Ereignisse vorgibt. Mit anderen Worten: Es grenzen immer nur solche Bereiche aneinander, in denen Ereignisse unterschiedlich bewertet werden. Alternativ kann sich der weitere Bereich mit dem ersten Bereich und dem zweiten Bereich überschneiden oder überlappen, oder er kann an einen der beiden Bereiche angrenzen.

Der mindestens eine weitere Bereich hat vorzugsweise eine rechteckige, quadratische, elliptische oder kreisförmige Form. Die Form und/oder die Lage des mindestens einen weiteren Bereiches kann im Wesentlichen frei definiert werden. Das heisst, dass eine Bedienungsperson mittels einer Eingabeeinheit die Form und/oder die Lage des weiteren Bereiches im Ereignisfeld nach Belieben wählen kann und dabei höchstens durch eine allfällig diskrete Auflösung der Eingabeeinheit oder einer Ausgabeeinheit, auf welcher der weitere Bereich allenfalls dargestellt wird, beschränkt ist.

Die Vorzüge der Erfindung kommen besonders dann zur Geltung, wenn das Garn ein Effektgarn ist, der erste Bereich zulässige Ereignisse für Stege des Effektgarns vorgibt und der mindestens eine dritte Bereich zulässige Ereignisse für Effekte des Effektgarns vorgibt. Das Effektgarn kann vorgängig charakterisiert werden, bspw. indem die darin vorkommenden Effektdurchmesser und Effektlängen bestimmt werden, und die Bereiche für die Ausreinigung von Fehlern können aufgrund der vorgängigen Charakterisierung definiert werden. So werden unerwünschte Fehler aus dem Effektgarn ausgereinigt, nicht jedoch die Effekte, die im Effektgarn verbleiben müssen.

Die unzulässigen Ereignisse werden vorzugsweise aus dem Garn entfernt, bspw. mittels einer Schneideinrichtung herausgeschnitten. Alternativ können die unzulässigen Ereignisse registriert werden, ohne dass sie aus dem Garn entfernt werden. Auch eine Kombination der beiden Alternativen, d. h. ein Entfernen und Registrieren der unzulässigen Ereignisse, ist möglich.

Die erfindungsgemässe Vorrichtung zur Ausreinigung von Fehlern aus einem in einer Textilmaschine, bspw. einer Spinnmaschine oder einer Spulmaschine, entlang seiner Längsrichtung bewegten Garn beinhaltet eine Steuereinheit zur Vorgabe eines Ereignisfeldes. Das Ereignisfeld ist ein Quadrant oder ein Teil eines Quadranten eines zweidimensionalen kartesischen Koordinatensystems, dessen Abszisse eine Erstreckung von Garnparameterwerten in der Längsrichtung und dessen Ordinate eine Abweichung des Garnparameters von einem Sollwert angibt. Das Ereignisfeld ist in Bereiche aufgeteilt, von denen ein erster Bereich, der an die Abszisse sowie an die Ordinate angrenzt und ferner durch eine Reinigungsgrenze begrenzt ist, zulässige Ereignisse und ein zum ersten Bereich komplementärer zweiter Bereich unzulässige Ereignisse vorgibt. Die Vorrichtung beinhaltet ausserdem eine Abtasteinheit zur Erfassung von Messwerten einer Eigenschaft des Garns entlang der Längsrichtung des Garns. Eine mit der Steuereinheit sowie mit der Abtasteinheit verbundene Auswerteeinheit dient zur Speicherung des Ereignisfeldes, zur Ermittlung von Werten eines Garnparameters aus den Messwerten und zur Klassierung der Werte des Garnparameters mitsamt ihrer Erstreckung in der Längsrichtung in dem Ereignisfeld. Die Steuereinheit ist zur Vorgabe eines Ereignisfeldes eingerichtet, in welchem mindestens ein weiterer Bereich definierbar ist, der im ersten Bereich unzulässige Ereignisse oder im zweiten Bereich zulässige Ereignisse vorgibt.

Die erfindungsgemässe Vorrichtung wird vorzugsweise zur Ausreinigung von Fehlern aus einem Effektgarn verwendet.

Die erfindungsgemässe Textilmaschine, bspw. Spinnmaschine oder Spulmaschine, beinhaltet eine erfindungsgemässe Vorrichtung zur Ausreinigung von Fehlern aus einem in der Textilmaschine entlang seiner Längsrichtung bewegten Garn.

Gemäss der vorliegenden Erfindung kann das Ereignisfeld auf einer Ausgabeeinheit dargestellt werden. Nötig ist eine solche Darstellung jedoch nicht, denn die Erfindung kommt auch ohne sie aus. Die erfindungsgemässe Bereitstellung des Ereignisfeldes, die Aufteilung des Ereignisfeldes in verschiedene Bereiche und die Klassierung der Ereignisse im Ereignisfeld können rein virtuell erfolgen.

Die Erfindung bietet den Vorteil einer grossen Flexibilität bei der Garnreinigung. Die Bereiche für zulässige und unzulässige Ereignisse können nach Belieben eingestellt und an das zu reinigende Garn angepasst werden. Die Reinigung von Effektgarn, auch von solchem mit unterschiedlich ausgebildeten Effekten, z. B. mit verschiedenen Effektdurchmessern und/oder verschiedenen Effektlängen, ist möglich. Zu lange und zu kurze Effekte werden als Fehler erkannt und nötigenfalls ausgereinigt. Bei alldem werden aber echte Effekte nicht als vermeintliche Fehler behandelt.

### AUFZÄHLUNG DER ZEICHNUNGEN

Nachfolgend wird die Erfindung anhand der Zeichnungen detailliert erläutert. Dabei wird hauptsächlich auf das bevorzugte Ausführungsbeispiel der Ausreinigung von Effektgarn Bezug genommen. Dieses Beispiel soll jedoch keineswegs die Allgemeinheit der Erfindung einschränken. Die Erfindung ist auch für andere Garnarten als Effektgarn verwendbar.
- Figur 1: zeigt schematisch eine erfindungsgemässe Spulmaschine mit einer erfindungsgemässen Vorrichtung.
- Figuren 2-5: zeigen verschiedene Ausführungsformen von Ereignisfeldern gemäss der Erfindung.

### AUSFÜHRUNG DER ERFINDUNG

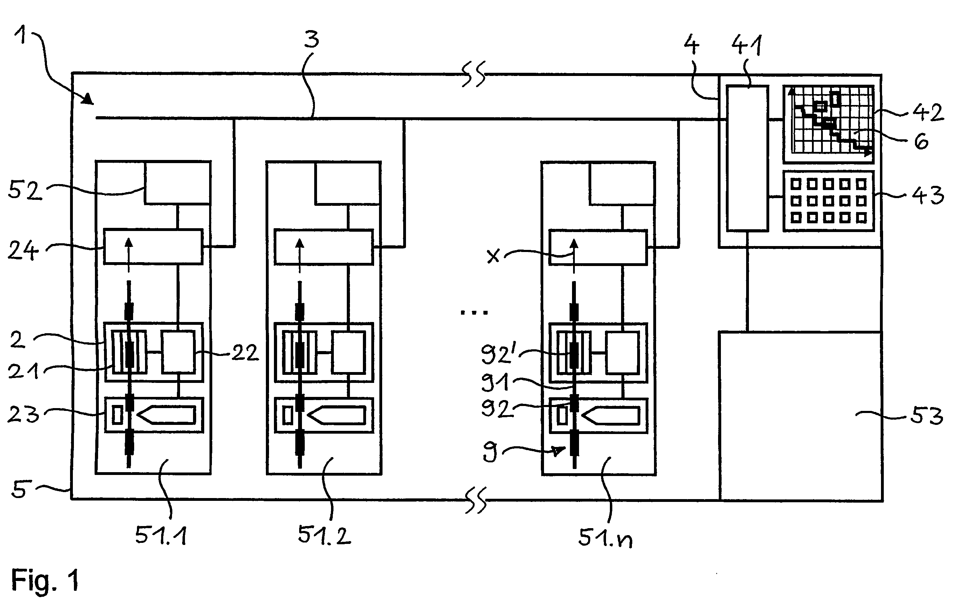

In **Figur 1** ist sehr schematisch eine erfindungsgemässe Spulmaschine 5 mit mehreren Spulstellen 51.1, 51.2, ..., 51.n dargestellt. Eine erfindungsgemässe Vorrichtung 1 ist in die Spulmaschine 5 eingebaut. An jeder Spulstelle 51.1, 51.2, ..., 52.n wird während des Umspulvorgangs Garn 9 von einem Messkopf 2 der erfindungsgemässen Vorrichtung 1 überwacht.

Der Messkopf 2 beinhaltet eine Abtasteinheit 21 zum Abtasten eines in Längsrichtung x bewegten Effektgarnes 9 mit Stegen 91 und Effekten 92, 92'. Unter dem Begriff "Abtasten" wird hier die sequenzielle Aufnahme einer Vielzahl von Messwerten an verschiedenen, vorzugsweise äquidistanten Stellen des Effektgarns 9 verstanden. Derartige Abtasteinheiten 21 sind an sich bekannt und brauchen hier deshalb nicht näher erläutert zu werden. Die Abtasteinheit 21 kann mit einem kapazitiven, optischen oder anderen Sensor ausgerüstet sein; es können auch mehrere gleiche oder verschiedene Sensoren innerhalb der Abtasteinheit 21 angeordnet sein. Die Abtasteinheit 21 kann mit Auswertemitteln für eine Vorauswertung der Messdaten ausgestattet sein. Sie gibt auf einer Datenleitung ein vorzugsweise elektrisches Ausgangssignal aus, das ein Mass für die Masse, die Dicke oder andere Eigenschaften des Effektgarnes 9 ist. Die Datenleitung führt zu einer Auswerteeinheit 22, die dazu eingerichtet ist, aus den Messwerten einen Garnparameter, z. B. die Garnmasse pro Längeneinheit, zu ermitteln.. Zu diesem Zweck beinhaltet die Auswerteeinheit 22 geeignete analoge und/oder digitale Auswertemittel, z. B. einen Mikroprozessor oder einen digitalen Signalprozessor (digital signal processor, DSP). Sie kann auch weitere Mittel wie Speichermittel zum Speichern von Daten beinhalten. Die Auswerteeinheit 22 kann als eigenständige Baugruppe realisiert oder in einem gemeinsamen Messkopfgehäuse mit der Abtasteinheit 21, vorzugsweise auf einer einzigen Leiterplatte, integriert sein.

Vorzugsweise ist an jeder Spulstelle 51.1, 51.2, ..., 51.n auch eine Schneideinrichtung 23 angebracht. Mit dieser werden Garnabschnitte mit unzulässigen Fehlern aus dem Effektgarn 9 herausgeschnitten. Die entsprechenden Schneidbefehle erhält die Schneideinrichtung 23 von der Auswerteeinheit 22. Die Schneideinrichtung 23 kann als eigenständige Baugruppe realisiert oder in einem gemeinsamen Messkopfgehäuse mit der Abtasteinheit 21 und /oder der Auswerteeinheit 22 untergebracht sein.

Der Messkopf 2 ist über einen Schnittstellenwandler 24 mit einer zentralen Steuereinheit 4 der erfindungsgemässen Vorrichtung 1 verbunden. Über die Verbindung wird der Messkopf 2 von der Steuereinheit 4 eingestellt und gesteuert, und der Messkopf 2 übermittelt Daten wie die ermittelten Garnparameter an die Steuereinheit 4. Eine Verbindungsleitung 3 zwischen allen Schnittstellenwandlern 24 und der Steuereinheit 4 kann als serieller Bus wie z. B. RS-485 ausgebildet sein. Der Schnittstellenwandler 24 kann zusätzlich auch mit einem Spulstellenrechner 52 der Spulstelle 51.1, 51.2, ..., 51.n verbunden sein. Die Steuereinheit 4 ist mit einem Zentralrechner 41 ausgestattet, welcher einerseits der Sammlung und weiteren Verarbeitung der von den Messköpfen 2 stammenden Messdaten, andererseits der Einstellung der Messköpfe 2 dient. Ferner weist die Steuereinheit 4 eine Ausgabeeinheit 42 und eine Eingabeeinheit 43 für eine Bedienungsperson auf. Die Ausgabeeinheit 42 dient der Ausgabe von Messdaten, von Resultaten der Auswertung und/oder der Veranschaulichung von eingegebenen Daten. Sie kann z. B. als Bildschirm und/oder Drucker ausgebildet sein. Die Eingabeeinheit 43 dient zum Eingeben von Daten durch die Bedienungsperson und insbesondere zur Eingabe und/oder zur Bearbeitung von (weiter unten erklärten) Bereichen eines Ereignisfeldes 6, welche zulässige bzw. unzulässige Ereignisse im Garn 9 vorgeben. Die Eingabeeinheit 43 kann z. B. eine Tastatur oder eine Computermaus beinhalten. Die Ausgabeeinheit 42 und die Eingabeeinheit 43 können gemeinsam als Sensorbildschirm (Touchscreen) ausgebildet sein. Die Ausgabeeinheit 42 und die Eingabeeinheit 43 sind mittels Datenleitungen mit dem Zentralrechner 41 verbunden. Es kann somit gesagt werden, dass die Auswerteeinheit 22 zumindest indirekt ebenfalls mit der Ausgabeeinheit 42 und der Eingabeeinheit 43 verbunden ist.

Die Steuereinheit 4 ist mit einem Steuerrechner 53 der Spulmaschine 5 verbunden. Statt in der Steuereinheit 4 können der Zentralrechner, 41, die Ausgabeeinheit 42 und/oder die Eingabeeinheit 43 in der Spulmaschine 5, z. B. im Steuerrechner 53, eingebaut sein. Die Datenleitungen können als Kabel oder drahtlos realisiert sein.

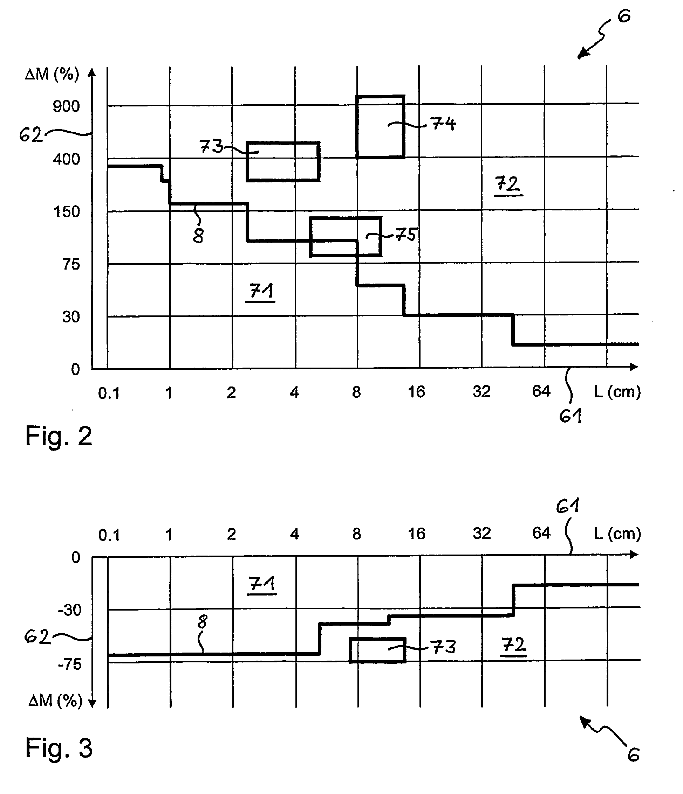

**Figur 2** zeigt eine an sich bekannte Klassiermatrix oder ein Ereignisfeld 6, in welchem längs einer Ordinate 62 Werte für positive Abweichungen eines Garnparameters von einem Sollwert in Prozenten und längs einer Abszisse 61 die Erstreckung oder Ausdehnung in Längsrichtung x oder Länge von Garnparameterabweichungen ist. Der Garnparameter kann z. B. eine Garnmasse M pro Längeneinheit oder ein Garndurchmesser sein. Die Garnparameterabweichungen werden in diesem Beispiel in Prozenten angegeben, wobei der Sollwert den Wert 0 % hat; andere, z. B. absolute Einheiten sind möglich. Die Abszisse 61 gibt an, über welche Länge L sich eine bestimmte Garnparameterabweichung ΔM erstreckt. Beide Achsen 61, 62 sind in diesem Beispiel zumindest annähernd logarithmisch, was aber nicht notwendig ist. Durch vertikale und horizontale Linien werden im Ereignisfeld 6 rechteckige Felder gebildet, die allgemein als Klassen bezeichnet werden. In diesem Ereignisfeld können Garnereignisse, d. h. gemessene Garnparameterabweichungen ΔM und ihre Längen L, als Punkte eingetragen werden. Bei vielen Ereignissen ergibt sich so eine Punktewolke (scatter plot).

Zusätzlich ist im Ereignisfeld 6 von Figur 2 eine Reinigungsgrenze 8 eingetragen. Die Reinigungsgrenze 8 teilt das Ereignisfeld in zwei Bereiche 71, 72 auf: einen ersten Bereich 71, in dem Abweichungen toleriert werden, und einen zweiten Bereich 72, der zum ersten Bereich 71 komplementär ist und in dem Abweichungen herausgeschnitten oder zumindest als unzulässige Fehler registriert werden. Die Reinigungsgrenze 8 gibt also an, wie weit ein Ereignis gegebener Länge vom Sollwert abweichen darf, bzw. wie lang ein Ereignis mit gegebener Abweichung sein darf, um gerade noch toleriert zu werden. Die Reinigungsgrenze 8 ist in diesem Ausführungsbeispiel eine treppenförmige Kurve, die horizontale und vertikale Abschnitte aufweist.

Im zweiten, unzulässigen Bereich 72 ist nun ein weiterer, dritter Bereich 73 eingezeichnet. Der dritte Bereich 73 gibt nun wiederum zulässige Ereignisse vor. Man kann sagen, dass im dritten Bereich 73 die Ausreinigung von Garnfehlern aufgehoben ist. Der dritte Bereich 73 ist in diesem Ausführungsbeispiel rechteckig und vom zweiten Bereich 72 vollständig umschlossen. Dasselbe gilt für einen weiteren, vierten Bereich 74. Ein weiterer, fünfter Bereich 75, der ebenfalls zulässige Ereignisse vorgibt, überlappt hingegen mit dem ersten Bereich 71. Der dritte Bereich 73, der vierte Bereich 74 und der fünfte Bereich 75 können z. B. erwünschte Effekte 92, 92' eines Effektgarns 9 (siehe Figur 1) davor bewahren, als vermeintliche Garnfehler herausgeschnitten zu werden.

Ein anderes Ereignisfeld 6 ist in **Figur 3** dargestellt. Es ist analog zum Ereignisfeld von Figur 2, nur sind hier negative Abweichungen ΔM des Garnparameters M gegenüber der Länge L aufgetragen, also z. B. *Garnmassen M* pro Längeneinheit oder Garndurchmesser, die kleiner sind als ein Sollwert 0. Auch in diesem Ereignisfeld 6 ist zunächst eine Reinigungsgrenze 8 eingezeichnet, die angibt, wie weit die negative Parameterabweichung ΔM zulässig ist. Ereignisse in einem ersten Bereich 71 sind zulässig, Ereignisse in einem zweiten Bereich 72 hingegen nicht. Ein dritter, bspw. rechteckiger Bereich 73 gibt Ereignisse vor, die innerhalb des zweiten Bereichs 72 toleriert werden sollen.

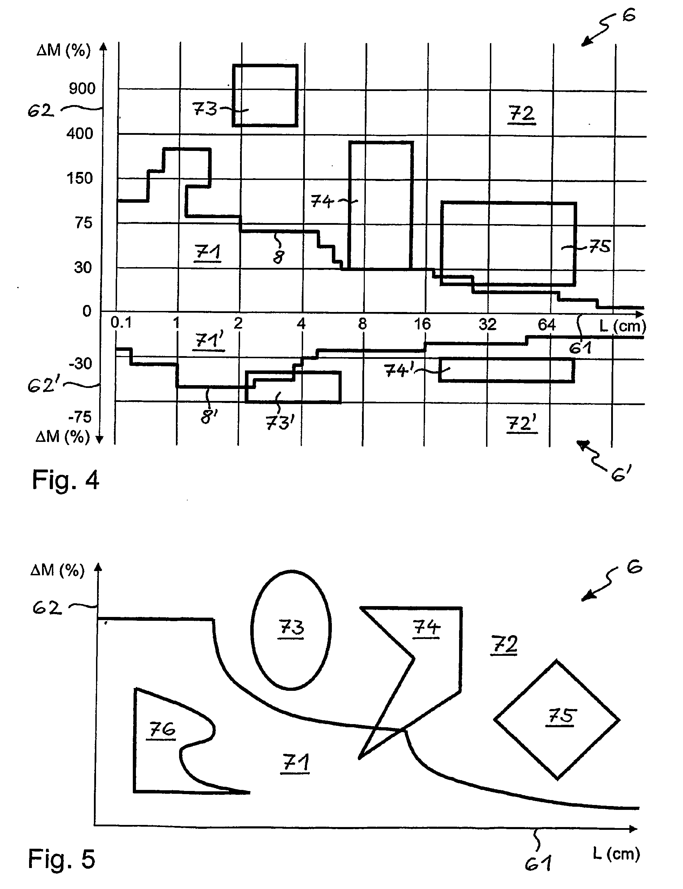

Bisweilen ist eine grafische Darstellung auzutreffen, in welcher die Ereignisfelder 6 der Figuren 2 und 3 derart zusammengesetzt sind, dass ihre Längenachsen 61 zusammenfallen und ihre Ordinaten 62 zusammen eine sich von negativen zu positiven Werten erstreckende Skala für die Abweichung ΔM aufweisen. Eine solche Darstellung zeigt in **Figur 4****.** Trotzdem handelt es sich bei einer solchen zusammengesetzten Darstellung immer noch um zwei Ereignisfelder 6, 6' gemäss der in dieser Schrift verwendeten Definition. Dies deshalb, weil es sich bei den zwei Ereignisfeldern 6, 6' um verschiedene Quadranten des Koordinatensystems handelt, in denen jeweils Abweichungen ΔM von einem Sollwert 0 in verschiedene Richtungen (positive bzw. negative Abweichungen) eingetragen sind. Jedes der beiden Ereignisfelder 6, 6' ist durch eine Reinigungsgrenze 8 in zwei zueinander komplementäre Bereiche 71, 71' bzw. 72, 72' für zulässige bzw. unzulässige Ereignisse aufgeteilt. Zusätzlich sind in den zweiten Bereichen 72, 72' weitere Bereiche 73, 73', 74, 74', 75 definiert, die darin zulässige Ereignisse vorgeben. Die weiteren Bereiche 73, 74' sind vom jeweiligen zweiten Bereich 72 bzw. 72' vollständig umschlossen; die weiteren Bereiche 75, 73' überschneiden sich mit dem jeweiligen ersten Bereich 71 bzw. 71' und dem jeweiligen zweiten Bereich 72 bzw. 72'; der weitere Bereich 74 grenzt an den ersten Bereich 71 an.

**Figur 5** gibt einige weitere Beispiele für Formen von Bereichen in einem Ereignisfeld 6 an. Ein erster Bereich 71 kann in zwei Richtungen durch die das Ereignisfeld 6 aufspannenden Achsen 61, 62 und durch eine stetige und/oder stufenförmige Kurve 8 definiert sein und zulässige Ereignisse vorgeben. Ein zweiter Bereich 72 kann im Wesentlichen komplementär zum ersten Bereich 71 sein und unzulässige Ereignisse vorgeben. Ein dritter, vom zweiten Bereich 72 vollständig umschlossener Bereich 73 kann elliptisch oder kreisförmig sein. Ein vierter, mit dem ersten Bereich 71 überlappender Bereich 74 kann ein Polygon mit einer oder mehreren konkaven Ecken sein. Ein fünfter, vom zweiten Bereich 72 vollständig umschlossener Bereich 75 kann ein konvexes Polygon sein. Ein sechster, vom ersten Bereich 72 vollständig umschlossener Bereich 76 kann von sowohl Geraden als auch Kurven beliebiger Form begrenzt sein. Der dritte Bereich 73, der vierte Bereich 74 und der fünfte Bereich 75 geben zulässige Ereignisse innerhalb des unzulässigen Bereichs 72 vor, während der sechste Bereich 76 unzulässige Ereignisse innerhalb des zulässigen Bereichs 71 vorgibt.

Nachfolgend wir die Wirkungsweise des erfindungsgemässen Verfahrens und der erfindungsgemässen Vorrichtung 1 anhand der Figuren 1 und 2 erläutert. Um beispielsweise ein textiles Effektgarn 9 (siehe Figur 1) zu reinigen, d. h. darin enthaltene Fehlstellen herauszuschneiden, wird es in an sich bekannter Weise durch die Abtasteinheit 2 in Richtung x hindurchbewegt. Dabei werden über die Datenleitung 33 laufend Werte für mindestens einen Parameter wie z. B. die Masse M pro Längeneinheit oder den Durchmesser des Effektgarns 9 an die Auswerteeinheit 22 abgegeben. In dieser ist ein Programm gespeichert und ausführbar, das die ermittelten Garnparameter gegebenenfalls speichert und laufend ihre Lage im Ereignisfeld 6 bestimmt. Es ist damit zu rechnen, dass Ereignisse, die zu Effekten 92, 92' gehören, jenseits der Reinigungsgrenze 8, d. h. im zweiten Bereich 72 (siehe Figur 2) liegen würden. Hier soll nun eine weitere Unterscheidung erfolgen, indem Ereignisse, die im dritten Bereich 73, im vierten Bereich 74 oder im fünften Bereich 75 liegen, als zulässige Effekte 92, 92' im Effektgarn 9 belassen werden. Ereignisse im zweiten Bereich 72 ausserhalb dieser zulässigen Bereiche 73-75 werden hingegen als unzulässige Fehler erkannt und aus dem Effektgarn 9 herausgeschnitten. Dies ist deshalb möglich, weil zulässige Effekte 92, 92' begrenzte Massenabweichungen ΔM oder Durchmesserabweichungen von einem Sollwert 0 und auch begrenzte Längen L in Längsrichtung x aufweisen. Somit ist nicht jeder Effekt 92, 92' ein unzulässiger Fehler, und nicht jede Dickstelle ist ein zulässiger Effekt.

Die weiteren zulässigen Bereiche 73-75 können vorgängig bestimmt werden. Dazu können bekannte Verfahren zur Charakterisierung von Effektgarn verwendet werden, wie sie in den Veröffentlichungen WO-2007/056883 A2, WO-2007/056884 A2 oder WO-2007/056885 A2 offenbart sind. Das auszureinigende Effektgarn 9 kann also vorgängig mit jenen Verfahren charakterisiert werden, z. B. an einem Labortestgerät oder an der erfindungsgemässen Vorrichtung 1 selbst. Im letzteren wird Fall vorteilhafterweise zum Zweck der Charakterisierung die Schneidfunktion der Schneideinrichtung 23 zur Entfernung von Fehlstellen desaktiviert. Diese Charakterisierung ermittelt insbesondere die Lagen im Ereignisfeld 6 der im betreffenden Effektgarn 9 auftretenden Effekte 92, 92'. Die Lagen der Effekte 9 werden über die Eingabeeinheit 43 und den Zentralrechner 41 oder eine entsprechende Schnittstelle in die Auswerteeinheit 22 eingegeben. Aufgrund der Lagen der Effekte 92, 92' werden dann die weiteren zulässigen Bereiche 73-75 definiert, in denen Effekte 92, 92' nicht ausgereinigt werden sollen. Die Definition der weiteren zulässigen Bereiche 73-75 kann automatisch oder manuell erfolgen. Die weiteren zulässigen Bereiche 73-75 können beliebige Formen haben, wovon die einfachsten ein Rechteck, ein Quadrat, eine Ellipse und ein Kreis sind. Sie können mit den ermittelten Lagen der Effekte 92, 92' im Ereignisfeld 6 übereinstimmen oder alternativ leicht grösser oder kleiner sein. Es kann vorteilhaft sein, die Lagen der Effekte 92, 92' im Ereignisfeld 6 auf der Ausgabeeinheit 42 darzustellen und die weiteren zulässigen Bereiche 73-75 von einer Bedienungsperson mittels der Eingabeeinheit 43, z. B. einer Computermaus, frei definieren oder bearbeiten zu lassen, wobei diese ebenfalls auf der Ausgabeeinheit 42 dargestellt werden. So kann auch eine Erfahrung der Bedienungsperson in die Definition der weiteren zulässigen Bereiche 73-75 einfliessen.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Solche Varianten können z. B. Kombinationen der oben diskutierten Ausführungsformen sein.

### BEZUGSZEICHENLISTE

- 1: Vorrichtung

- 2: Messkopf
- 21: Abtasteinheit
- 22: Auswerteeinheit
- 23: Schneideinrichtung

- 3: Datenleitung

- 4: Steuereinheit
- 41: Zentralrechner
- 42: Ausgabeeinheit
- 43: Eingabeeinheit

- 5: Spulmaschine
- 51.1, 51.2, ..., 51.n: Spulstellen
- 52: Spulstellenrechner
- 53: Steuerrechner

- 6, 6': Ereignisfeld
- 61: Abszisse
- 62, 62': Ordinate

- 71, 71': erster, "zulässiger" Bereich
- 72, 72': zweiter, "unzulässiger" Bereich
- 73, 73', 74, 74', 75: weitere, "zulässige" Bereiche
- 76: weiterer, "unzulässiger" Bereich

- 8, 8': Reinigungsgrenze

- 9: Effektgarn
- 91: Steg
- 92, 92': Effekte

- x: Längs- und Bewegungsrichtung des Effektgarns

## Patentansprüche

1. Verfahren zur Ausreinigung von Fehlern aus einem in einer Textilmaschine, bspw.
einer Spinnmaschine oder einer Spulmaschine (5), entlang seiner Längsrichtung (x) bewegten Garn (9), wobei
Messwerte einer Eigenschaft des Garns (9) entlang der Längsrichtung (x) des Garns (9) erfasst werden,
Werte eines Garnparameters (M) aus den Messwerten ermittelt werden,
ein Ereignisfeld (6) bereitgestellt wird,
das ein Quadrant oder ein Teil eines Quadranten eines zweidimensionalen kartesischen Koordinatensystems ist, dessen Abszisse (61) eine Erstreckung (L) von Garnparameterwerten in der Längsrichtung (x) und dessen Ordinate (62) eine Abweichung (ΔM) des Garnparameters (M) von einem Sollwert angibt, und
das in Bereiche (71, 72) aufgeteilt wird, von denen
ein erster Bereich (71), der an die Abszisse (61) sowie an die Ordinate (62) angrenzt und ferner durch eine Reinigungsgrenze (8) begrenzt ist, zulässige Ereignisse und
ein zum ersten Bereich (71) komplementärer zweiter Bereich (72) unzulässige Ereignisse
vorgibt, und
die Werte des Garnparameters (M) mitsamt ihrer Erstreckung (L) in der Längsrichtung (x) in dem Ereignisfeld (6) klassiert werden,
**dadurch gekennzeichnet,**
**dass** im Ereignisfeld (6) mindestens ein weiterer Bereich (73-76) definiert wird, der im ersten Bereich (71) unzulässige Ereignisse oder im zweiten Bereich (72) zulässige Ereignisse vorgibt.

2. Verfahren nach Anspruch 1, wobei der weitere Bereich (73-76) vom ersten Bereich (71) oder vom zweiten Bereich (72) im Wesentlichen umschlossen ist.

3. Verfahren nach Anspruch 1, wobei sich der weitere Bereich (75) mit dem ersten Bereich (71) und dem zweiten Bereich (72) überschneidet oder an einen der beiden Bereiche (71, 72) angrenzt.

4. Verfahren nach einem der vorangehenden Ansprüche, wobei der mindestens eine weitere Bereich (73-75) rechteckig, quadratisch, elliptisch oder kreisförmig ist.

5. Verfahren nach einem der vorangehenden Ansprüche, wobei die Form und/oder die Lage des mindestens einen weiteren Bereiches (73-76) im Wesentlichen frei definiert wird.

6. Verfahren nach einem der vorangehenden Ansprüche, wobei das Garn (9) ein Effektgarn ist, der erste Bereich (71) zulässige Ereignisse für Stege (91) des Effektgarns (9) vorgibt und der mindestens eine dritte Bereich (73-75) zulässige Ereignisse für Effekte (92, 92') des Effektgarns (9) vorgibt.

7. Verfahren nach Anspruch 6, wobei das Effektgarn (9) vorgängig charakterisiert wird und Bereiche (73-76) für die Ausreinigung von Fehlern aufgrund der vorgängigen Charakterisierung definiert werden.

8. Verfahren nach einem der vorangehenden Ansprüche, wobei die unzulässigen Ereignisse aus dem Garn (9) entfernt werden.

9. Vorrichtung (1) zur Ausreinigung von Fehlern aus einem in einer Textilmaschine, bspw. einer Spinnmaschine oder einer Spulmaschine (5), entlang seiner Längsrichtung (x) bewegten Garn (9), beinhaltend
eine Steuereinheit (4) zur Vorgabe eines Ereignisfeldes (6),
das ein Quadrant oder ein Teil eines Quadranten eines zweidimensionalen kartesischen Koordinatensystems ist, dessen Abszisse (61) eine Erstreckung (L) von Garnparameterwerten in der Längsrichtung (x) und dessen Ordinate (62) eine Abweichung (ΔM) des Garnparameters (M) von einem Sollwert angibt, und
das in Bereiche (71, 72) aufgeteilt ist, von denen
ein erster Bereich (71), der an die Abszisse (61) sowie an die Ordinate (62) angrenzt und ferner durch eine Reinigungsgrenze (8) begrenzt ist, zulässige Ereignisse und
ein zum ersten Bereich (71) komplementärer zweiter Bereich (72) unzulässige Ereignisse
vorgibt,
eine Abtasteinheit (2) zur Erfassung von Messwerten einer Eigenschaft des Garns (9) entlang der Längsrichtung (x) des Garns (9) und
eine mit der Steuereinheit (4) sowie mit der Abtasteinheit (2) verbundene Auswerteeinheit (22)
zur Speicherung des Ereignisfeldes (6),
zur Ermittlung von Werten eines Garnparameters (M) aus den Messwerten und
zur Klassierung der Werte des Garnparameters (M) mitsamt ihrer Erstreckung (L) in der Längsrichtung (x) in dem Ereignisfeld (6),
**dadurch gekennzeichnet,**
**dass** die Steuereinheit (4) zur Vorgabe eines Ereignisfeldes (6) eingerichtet ist, in welchem mindestens ein weiterer Bereich (73-76) definierbar ist, der im ersten Bereich (71) unzulässige Ereignisse oder im zweiten Bereich (72) zulässige Ereignisse vorgibt.

10. Vorrichtung (1) nach Anspruch 9, wobei die Steuereinheit eine Anzeigeeinheit (42) zur Anzeige des Ereignisfeldes (6) beinhaltet.

11. Vorrichtung (1) nach Anspruch 9 oder 10, wobei die Steuereinheit (4) eine Eingabeeinheit (5) zur Eingabe und/oder Bearbeitung der Bereiche (71-76) beinhaltet.

12. Vorrichtung (1) nach einem der Ansprüche 9-11, wobei die Vorrichtung (1) eine Schneideinrichtung (23) zur Entfernung von unzulässigen Ereignissen aus dem Garn (9) beinhaltet.

13. Verwendung der Vorrichtung (1) nach einem der Ansprüche 9-12 zur Ausreinigung von Fehlern aus einem Effektgarn (9).

14. Textilmaschine (5), bspw. Spinnmaschine oder Spulmaschine, mit einer Vorrichtung (1) zur Ausreinigung von Fehlern aus einem in der Textilmaschine (5) entlang seiner Längsrichtung (x) bewegten Garn (9),
**dadurch gekennzeichnet,**
**dass** die Vorrichtung (1) eine Vorrichtung nach einem der Ansprüche 9-12 ist.

## Claims

1. A method for clearing out defects from a yarn (9) moved in a textile machine, e.g. a spinning machine or a winding machine (5), along its longitudinal direction (x), wherein
measured values of a property of the yarn (9) are detected along the longitudinal direction (x) of the yarn,
values of a yarn parameter (M) are determined from the measured values,
an event field (6) is provided
which is a quadrant, or a part of a quadrant, of a two-dimensional Cartesian coordinate system, the abscissa (61) of which states an extension (L) of yarn parameter values in the longitudinal direction (x) and the ordinate (62) of which indicates a deviation (ΔM) of the yarn parameter (M) from a target value, and
which field is divided into areas (71, 72), of which
a first area (71), which is adjacent to the abscissa (61) and the ordinate (62) and is further delimited by a clearing limit (8), predetermines permissible events and
a second area (72) which is complementary to the first area (71) predetermines impermissible events, and
the values of the yarn parameter (M), including its extension (L) in the longitudinal direction (x), are classified in the event field (6),
**characterized in that**
at least one further area (73 to 76) is defined in the event field (6) which predetermines impermissible events in the first area (71) or permissible events in the second area (72).

2. The method according to claim 1, wherein the further area (73 to 76) is substantially enclosed by the first area (71) or the second area (72).

3. The method according to claim 1, wherein the further area (75) intersects with the first area (71) and the second area (72) or is adjacent to one of the two areas (71, 72).

4. The method according to one of the preceding claims, wherein the at least one further area (73-75) is rectangular, square, elliptical or circular.

5. The method according to one of the preceding claims, wherein the shape and/or the position of the at least one further area (73 to 76) is defined in a substantially free manner.

6. The method according to one of the preceding claims, wherein the yarn (9) is a fancy yam, the first area (71) predetermines permissible events for base yarns (91) of the fancy yarn (9) and the at least one third area (73 to 75) predetermines permissible events for slubs (92, 92') of the fancy yarn (9).

7. The method according to claim 6, wherein the fancy yarn (9) is characterized beforehand and areas (73 to 76) for clearing out defects are defined on the basis of the preceding characterization.

8. The method according to one of the preceding claims, wherein the impermissible events are removed from the yarn (9).

9. An apparatus (1) for clearing out defects from a yarn (9) moved in a textile machine, e.g. a spinning machine or a winding machine (5), along its longitudinal direction (x), containing
a control unit (4) for predetermining an event field (6)
which is a quadrant, or a part of a quadrant, of a two-dimensional Cartesian coordinate system, the abscissa (61) of which states an extension (L) of yarn parameter values in the longitudinal direction (x) and the ordinate (62) of which indicates a deviation (ΔM) of the yarn parameter (M) from a target value, and which field is divided into areas (71, 72), of which
a first area (71), which is adjacent to the abscissa (61) and the ordinate (62) and is further delimited by a clearing limit (8), predetermines permissible events and
a second area (72) which is complementary to the first area (71) predetermines impermissible events,
a scanning unit (2) for detecting the measured values of a property of the yarn (9) along the longitudinal direction (x) of the yarn (9) and
an evaluation unit (22) connected with the control unit (4) and the scanning unit (2)
for storing the event field (6),
for determining values of a yarn parameter (M) from the measured values, and
for classifying the values of the yarn parameter (M) including its extension (L) in the longitudinal direction (x) in the event field (6),
**characterized in that**
the control unit (4) is set up for predetermining an event field (6) in which at least one further area (73 to 76) can be defined which predetermines impermissible events in the first area (71) or permissible events in the second area (72).

10. The apparatus (1) according to claim 9, wherein the control unit contains a display unit (42) for displaying the event field (6).

11. The apparatus (1) according to claim 9 or 10, wherein the control unit (4) contains an input unit (5) for entering and/or editing the areas (71 to 76).

12. The apparatus (1) according to one of the claims 9 to 11, wherein the apparatus (1) contains a cutting device (23) for removing impermissible events from the yarn (9).

13. The use of the apparatus (1) according to one of the claims 9 to 12 for clearing out defects from a fancy yarn (9).

14. A textile machine (5), such as a spinning machine or winding machine for example, with apparatus (1) for clearing out defects from a yarn (9) moved the textile machine (5) along its longitudinal direction (x),
**characterized in that**
the apparatus (1) is an apparatus according to one of the claims 9 to 12.

## Revendications

1. Procédé pour éliminer des défauts d'un filé (9) déplacé dans le sens de sa longueur (x) dans une machine textile, par exemple un métier à filer ou une bobineuse (5), dans lequel
des valeurs de mesure d'une propriété du filé (9) sont détectées dans le sens de la longueur (x) du filé (9),
des valeurs d'un paramètre du filé (M) sont déterminées à partir des valeurs de mesure,
un champ d'événements (6) est créé,
qui est un quadrant ou une partie d'un quadrant d'un système de coordonnées cartésien à deux dimensions dont l'abscisse (61) indique une étendue (L) de valeurs de paramètre du filé dans le sens de la longueur (x) et l'ordonnée (62) un écart (ΔM) du paramètre du filé (M) par rapport à une valeur de consigne, et
qui est partagé en zones (71, 72) parmi lesquelles
une première zone (71) contiguë à l'abscisse (61) ainsi qu'à l'ordonnée (62) et délimitée par une limite de nettoyage (8) détermine les événements admissibles et
une deuxième zone (72) complémentaire de la première zone (71) détermine les événements non admissibles,
et
les valeurs du paramètre du filé (M) sont classées avec leur étendue (L) dans le sens de la longueur (x) dans le champ d'événements (6),
**caractérisé**
**en ce qu'**au moins une autre zone (73-76) est définie dans le champ d'événements (6), laquelle détermine des événements non admissibles dans la première zone (71) ou admissibles dans la deuxième zone (72).

2. Procédé selon la revendication 1, dans lequel l'autre zone (73-76) est pour l'essentiel entourée par la première zone (71) ou par la deuxième zone (72).

3. Procédé selon la revendication 1, dans lequel l'autre zone (75) se recoupe avec la première zone (71) et la deuxième zone (72) ou est limitrophe de l'une des deux zones (71, 72).

4. Procédé selon l'une des revendications précédentes, dans lequel l'au moins une autre zone (73-75) est rectangulaire, carrée, elliptique ou circulaire.

5. Procédé selon l'une des revendications précédentes, dans lequel la forme et/ou la position de l'au moins une autre zone (73-76) est pour l'essentiel définie librement.

6. Procédé selon l'une des revendications précédentes, dans lequel le filé (9) est un filé à effets, la première zone (71) détermine des événements admissibles pour les sections entre effets (91) du filet à effets (9) et l'au moins une troisième zone (73-75) détermine les événements admissibles pour les effets (92, 92') du filé à effets (9).

7. Procédé selon la revendication 6, dans lequel le filé à effets (9) est caractérisé au préalable et des zones (73-76) pour l'élimination de défauts sont définies sur la base de la caractérisation préalable.

8. Procédé selon l'une des revendications précédentes, dans lequel les événements non admissibles sont éliminés du filé (9).

9. Dispositif (1) pour l'élimination de défauts dans un filé (9) déplacé dans le sens de sa longueur (x) dans une machine textile, par exemple un métier à filer ou une bobineuse (5), comprenant
une unité de commande (4) pour déterminer un champ d'événements (6),
qui est un quadrant ou une partie d'un quadrant d'un système de coordonnées cartésien à deux dimensions dont l'abscisse (61) indique une étendue (L) de valeurs de paramètre du filé dans le sens de la longueur (x) et l'ordonnée (62) un écart (ΔM) du paramètre du filé (M) par rapport à une valeur de consigne, et
qui est partagé en zones (71, 72) parmi lesquelles
une première zone (71) contiguë à l'abscisse (61) ainsi qu'à l'ordonnée (62) et délimitée par une limite de nettoyage (8) détermine les événements admissibles et
une deuxième zone (72) complémentaire de la première zone (71) détermine les événements non admissibles,
une unité de balayage (2) pour l'acquisition de valeurs de mesure d'une propriété du filé (9) dans le sens de la longueur (x) du filé (9) et
une.unité d'analyse (22) reliée à l'unité de commande (4) et à l'unité de balayage (2)
pour enregistrer le champ d'événements (6),
pour déterminer des valeurs d'un paramètre du filé (M) à partir des valeurs de mesure et
pour classer les valeurs du paramètre du filé (M) avec leur étendue (L) dans le sens de la longueur (x) dans le champ d'événements (6),
**caractérisé**
**en ce que** l'unité de commande (4) est équipée pour déterminer un champ d'événements (6) dans lequel au moins une autre zone (73-76) peut être définie, laquelle détermine des événements non admissibles dans la première zone (71) ou des événements admissibles dans la deuxième zone (72).

10. Dispositif (1) selon la revendication 9, dans lequel l'unité de commande comprend une unité d'affichage (42) pour l'affichage du champ d'événements (6).

11. Dispositif (1) selon la revendication 9 ou 10, dans lequel l'unité de commande (4) comprend une unité de saisie (5) pour la saisie et/ou le traitement des zones (71-76).

12. Dispositif (1) selon l'une des revendications 9 à 11, lequel dispositif (1) comprend un dispositif de coupe (23) pour supprimer les événements non admissibles du filé (9).

13. Utilisation du dispositif (1) selon l'une des revendications 9 à 12 pour éliminer des défauts d'un filé à effets (9).

14. Machine textile (5), par exemple métier à filer ou bobineuse, avec un dispositif (1) pour l'élimination de défauts dans un filé (9) déplacé dans le sens de sa longueur (x) dans la machine textile (5), **caractérisée en ce que** le dispositif (1) est un dispositif selon l'une des revendications 9 à 12.
